(19) Europäisches Patentamt
European Patent Office
Office européen des brevets

(11) Veröffentlichungsnummer : **0 021 374**
**B1**

(12) # EUROPÄISCHE PATENTSCHRIFT

(45) Veröffentlichungstag der Patentschrift : **13.01.82**

(51) Int. Cl.³ : **C 07 C 85/06**, C 07 C 89/00, C 07 C 87/58, C 07 C 91/42

(21) Anmeldenummer : **80103485.1**

(22) Anmeldetag : **21.06.80**

(54) **Verfahren zur Herstellung von 4-Amino-diphenylaminen.**

(30) Priorität : **03.07.79 DE 2926714**

(43) Veröffentlichungstag der Anmeldung : **07.01.81 (Patentblatt 81/01)**

(45) Bekanntmachung des Hinweises auf die Patenterteilung : **13.01.82 Patentblatt 82/02**

(84) Benannte Vertragsstaaten : **AT BE CH DE FR GB IT LI NL SE**

(56) Entgegenhaltungen :
**CA - A - 1 006 183**
**DE - A - 2 026 053**
**DE - A - 2 307 233**
**FR - A - 1 179 775**
**GB - A - 282 111**

**CHEMICAL ABSTRACTS, Band 50, Nr. 5, 10. März 1956, Zusammenfassung Nr. 3336h, Columbus, Ohio, US,**
**N.S. KOZLOV et al. : « Catalytic amination of organic compounds. II. Catalytic amination of phenols »**

(73) Patentinhaber : **BAYER AG**
**Zentralbereich Patente, Marken und Lizenzen**
**D-5090 Leverkusen 1, Bayerwerk (DE)**

(72) Erfinder : **Wedemeyer, Karlfried, Dr.**
**Bilharzstrasse 7**
**D-5000 Köln 80 (DE)**
Erfinder : **Böhm, Siegfried, Dr.**
**Meisenweg 7**
**D-4047 Dormagen 1 (DE)**

**EP 0 021 374 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Verfahren zur Herstellung von 4-Amino-diphenylaminen

Die Erfindung betrifft ein Verfahren zur Herstellung von 4-Amino-diphenylaminen.

In der Technik sind verschiedene Möglichkeiten bekannt, 4-Amino-diphenylamine herzustellen. So kann man nach der US 2 927 943 und US 3 313 854 p-Nitro-chlor-benzol mit Anilin in Gegenwart von Kupfer-Katalysatoren zu dem p-Nitro-diphenylamin umsetzen und dieses zu dem p-Amino-diphenylamin reduzieren. Es ist auch möglich, entsprechend der DE-OS 2 654 936 Diphenylamin zu nitrosieren und das gebildete N-Nitroso-diphenylamin in salzsaurem Medium zu dem p-Nitroso-diphenylamin umzulagern. Die Reduktion der Nitrosogruppe in dem p-Nitroso-diphenylamin zur Aminogruppe kann entsprechend der DE-OS 2 355 737 mit Natriumsulfid erfolgen. Bei beiden Verfahrenswegen entstehen als Zwangsanfall mindestens stöchiometrische Mengen an anorganischen Salzen, die das Abwasser belasten.

Die Umsetzung von p-Hydroxy-diphenylamin mit Ammoniak zu den p-Amino-diphenylaminen in Gegenwart von Katalysatoren ist aus der CA 1 006 183 bekannt. Als Katalysatoren werden beispielsweise Nickel, Platin und Palladium genannt. Vor Gebrauch dieser Katalysatoren ist es erforderlich, sie mit Wasserstoff zu aktivieren. Hierbei müssen die bei der Arbeit mit Wasserstoff auftretenden sicherheitstechnischen aufwendigen Vorkehrungen getroffen werden. Die hier verwendeten Katalysatoren, die allgemein als Hydrierungs-Dehydrierungskatalysatoren bekannt sind, sind sehr empfindliche Systeme, die leicht vergiftbar sind und so an Aktivität verlieren (Ullmann, *1963*, Band 14, Seite 643, Ann. *565*, 51 (1948).

Es wurde ein Verfahren zur Herstellung von 4-Amino-diphenylaminderivaten gefunden, das dadurch gekennzeichnet ist, daß man in Gegenwart eines γ-Aluminiumoxid enthaltenden Katalysators Hydroxybenzolderivate der Formel

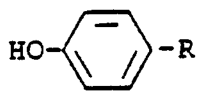

(I)

worin

R Hydroxy oder Amino bedeutet

mit einem Anilinderivat der Formel

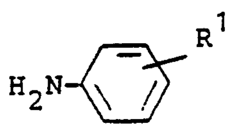

(II)

worin

$R^1$ für Wasserstoff oder einen Alkylrest steht,

umsetzt und anschließend am gleichen Katalysator mit Ammoniak behandelt.

Bei dem erfindungsgemäßen Verfahren entsteht als Zwischenprodukt ein Hydroxy-diphenylamin-Derivat der Formel

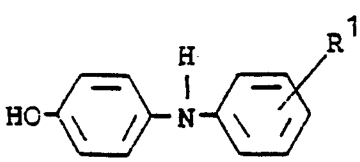

(III)

worin

$R^1$ die obengenannte Bedeutung hat,

das üblicherweise nicht isoliert wird. Es ist selbstverständlich aber auch möglich, das Hydroxy-diphenyl-amin-Derivat als Zwischenprodukt zu isolieren und es dann erfindungsgemäß mit Ammoniak in Gegenwart eines γ-Aluminiumoxid enthaltenden Katalysators umzusetzen.

Das erfindungsgemäße Verfahren kann durch die folgenden Reaktionsgleichungen erläutert werden :

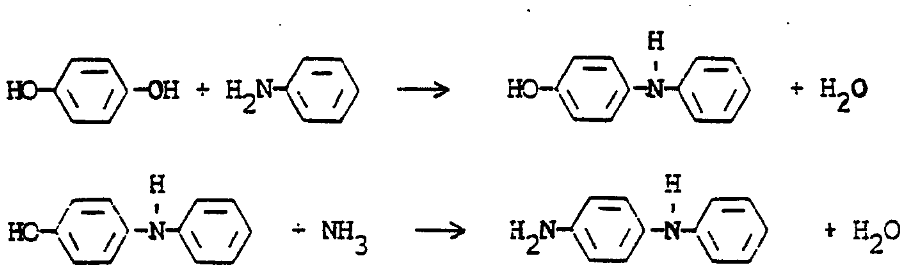

Ein Alkylrest ($R^1$) kann ein geradkettiger oder verzweigter aliphatischer Kohlenwasserstoffrest mit 1 bis etwa 12 Kohlenstoffatomen sein. Bevorzugt ist ein Niederalkylrest mit bis zu etwa 6 Kohlenstoffatomen. Beispielsweise seien die folgenden Alkylreste genannt: Methyl, Ethyl, Propyl, Isopropyl, Butyl, Isobutyl, Pentyl, Isopentyl, Hexyl und Isohexyl.

Hydroxybenzolderivate für das erfindungsgemäße Verfahren können beispielsweise Hydrochinon, oder p-Amino-phenol sein.

Hydroxybenzol-Derivate sind an sich bekannt (FR 1079454 und DRP 83433) und können beispielsweise durch Oxidation von p-Diisopropylbenzol mit Cumolhydroperoxid (Hydrochinon) oder durch Umlagerung von Phenylhydroxylamin mit Säuren (p-Aminophenol) hergestellt werden.

Anilinderivate für das erfindungsgemäße Verfahren können beispielsweise Anilin, o-, m- oder p-Methylanilin, o-, m- oder p-Ethylanilin, o-, m- oder p-Propylanilin, o-, m- oder p-Isopropylanilin, o-, m- oder p-Butylanilin, o-, m- oder p-Isobutylanilin, o-, m- oder p-Pentylanilin, o-, m- oder p-Isopentylanilin, o-, m- oder p-Hexylanilin oder o-, m- oder p-Isohexylanilin sein. Bevorzugte Anilinderivate sind Anilin, o-, m- oder p-Methylanilin.

Anilinderivate sind an sich bekannt (BIOS-Report Nr. 1157, S. 25) und können beispielsweise durch Reduktion von Nitrobenzolderivaten mit Eisenspänen hergestellt werden.

Das erfindungsgemäße Verfahren wird in Gegenwart von $\gamma$-Aluminiumoxid als Katalysator durchgeführt. $\gamma$-Aluminiumoxid ist an sich bekannt (Ullmann, *1974*, Band 7, Seite 298 ff) und kann beispielsweise durch Calcinieren von Aluminiumoxidhydraten bei erhöhter Temperatur hergestellt werden.

Der $\gamma$-Aluminiumoxid-Katalysator kann mit festen sauren Oxiden, wie Siliciumdioxid, Wolframtrioxid, Chrom(III)-oxid und/oder Phosphorpentoxid, dotiert sein. Bevorzugt wird für das erfindungsgemäße Verfahren ein undotiertes, Aluminiumoxid verwandt.

Die Menge des $\gamma$-Aluminiumoxids für das erfindungsgemäße Verfahren kann in weiten Grenzen schwanken, im allgemeinen ist es zweckmäßig, bei diskontinuierlicher Arbeitsweise 0,1 bis 10 Gew.-Teile $\gamma$-Aluminiumoxid bezogen auf 1 Gew.-Teil Hydroxybenzolderivat einzusetzen. Es ist bevorzugt 0,5 bis 5 Gew.-Teile $\gamma$-Aluminiumoxid bezogen auf 1 Gew.-Teil des Hydroxybenzolderivats einzusetzen.

Selbstverständlich kann der Katalysator bei der diskontinuierlichen Arbeitsweise mehrfach benutzt werden. Bei kontinuierlicher Arbeitsweise ordnet man den Katalysator vorzugsweise in einem Reaktionsrohr an und leitet das Reaktionsgemisch hindurch.

Das Mengenverhältnis Hydroxybenzolderivat zu Anilinderivat wird im wesentlichen durch die Stöchiometrie der Reaktion festgelegt. Es ist möglich, die Anilin-Komponente im Überschuß einzusetzen und so praktisch als Lösungsmittel zu verwenden.

Im allgemeinen kann man 1 bis 10 Mol des Anilinderivats, bezogen auf 1 Mol des Hydroxybenzolderivats einsetzen.

Bei der Verwendung von Hydrochinon ist es insbesondere bevorzugt, 1,05 bis 8 Mol des Anilinderivats bezogen auf 1 Mol des Hydrochinon einzusetzen.

Bei der Verwendung von 4-Aminophenol als Hydroxybenzolderivat ist es insbesondere bevorzugt 2 bis 8 Mol des Anilinderivates bezogen auf 1 Mol 4-Aminophenol einzusetzen.

Das Mengenverhältnis Ammoniak zu dem Hydroxybenzolderivat ist in weiten Grenzen variabel. Im allgemeinen wird man Ammoniak im Überschuß, beispielsweise von 10 bis 40 Mol, bezogen auf 1 Mol des Hydroxybenzolderivates, einsetzen.

Bei der Verwendung von Hydrochinon setzt man in der zweiten Reaktionsstufe bevorzugt 15 bis 30 Mol, insbesondere bevorzugt 18 bis 28 Mol, Ammoniak bezogen auf 1 Mol Hydrochinon ein.

Bei der Verwendung von 4-Aminophenol setzt man bevorzugt 15 bis 30 Mol, insbesondere bevorzugt 18 bis 28 Mol, Ammoniak, bezogen auf 1 Mol 4-Aminophenol, ein.

Das erfindungsgemäße Verfahren wird im allgemeinen im Temperaturbereich von 200 bis 450 °C durchgeführt.

Bei der Umsetzung eines Dihydroxy-Benzolderivates mit einem Anilinderivat führt man die Umsetzung bevorzugt im Temperaturbereich von 200 bis 350 °C, insbesondere bevorzugt von 220 bis 300 °C, durch und steigert nach Zugabe des Ammoniaks die Reaktionstemperatur bis zu dem Bereich von 300 bis 450 °C, bevorzugt von 320 bis 400 °C.

Bei der Verwendung eines Aminobenzol-Derivates führt man die gesamte Umsetzung bevorzugt im Temperaturbereich von 300 bis 450 °C, bevorzugt von 320 bis 400 °C, durch.

In einer besonders bevorzugten Ausführungsform des erfindungsgemäßen Verfahrens setzt man im Temperaturbereich von 230 bis 280 °C 1 bis 10 Mol Anilin pro Mol Hydrochinon um. Die weitere Umsetzung mit 15 bis 30 Mol Ammoniak wird in dieser bevorzugten Ausführungsform im Temperaturbereich von 330 bis 380 °C durchgeführt.

In einer weiteren bevorzugten Ausführungsform des erfindungsgemäßen Verfahrens setzt man im Temperaturbereich von 330 bis 380 °C 4-Amino-phenol mit Anilin um und behandelt das entstehende Gemisch aus 4-Hydroxy- und 4-Amino-diphenylamin im gleichen Temperaturbereich mit 15 bis 30 Mol Ammoniak.

Das erfindungsgemäße Verfahren wird im allgemeinen bei Überdruck durchgeführt. Zweckmäßigerweise kann man im Druckbereich von 1 bis 400 bar arbeiten. Bevorzugt für das erfindungsgemäße Verfahren ist der Druckbereich von 10 bis 350 bar.

Insbesondere bei einer diskontinuierlichen Arbeitsweise des erfindungsgemäßen Verfahrens führt

man die Umsetzung bevorzugt in einem Autoklaven durch. Hierbei wählt man zweckmäßigerweise den Druck als Reaktionsdruck, der sich bei der gewählten Reaktionstemperatur im geschlossenen Autoklaven einstellt.

Das erfindungsgemäße Verfahren kann in Gegenwart von Lösungsmitteln durchgeführt werden, die mit den Reaktionskomponenten nicht reagieren. Im allgemeinen ist es jedoch bevorzugt, daß erfindungsgemäße Verfahren im Überschuß der Anilinkomponente durchzuführen.

Das erfindungsgemäße Verfahren wird im allgemeinen als Eintopfverfahren ohne Isolierung des Hydroxy-diphenyl-aminderivats der Formel (III) als Zwischenprodukt durchgeführt. Falls man jedoch das Zwischenprodukt isoliert hat oder ein auf andere Weise hergestelltes Hydroxy-diphenylaminderivat der Formel (III) umsetzen möchte, kann selbstverständlich erfindungsgemäß verfahren werden.

Bei diskontinuierlicher Arbeitsweise können beispielsweise die Reaktionskomponenten und der Katalysator in einem Autoklaven bei der jeweiligen Reaktionstemperatur gerührt werden. Vorteilhafterweise rührt man das Hydroxybenzolderivat zunächst mit dem Katalysator und dem Anilinderivat bei der Reaktionstemperatur des ersten Reaktionsschrittes, pumpt dann Ammoniak ein und erwärmt auf die Reaktionstemperatur des zweiten Reaktionsschrittes. Das Reaktionsprodukt wird nach dem Abkühlen des Autoklaven in an sich bekannter Weise, z.B. durch Filtration, vom Katalysator abgetrennt und durch Destillation gereinigt.

Bei kontinuierlicher Arbeitsweise kann man beispielsweise das Hydroxybenzolderivat in dem Anilinderivat lösen und die Lösung durch ein mit γ-Aluminiumoxid-katalysator gefüllten Rohr leiten, das auf die jeweilige Reaktionstemperatur erwärmt wurde. Die Umsetzung kann sowohl in der Dampf- als auch in der Flüssigphase durchgeführt werden. Man kann das Hydroxybenzol/Anilinderivatgemisch sowohl von oben nach unten als auch von unten nach oben durch das Reaktionsrohr führen. In ähnlicher Weise kann die Umsetzung mit dem Ammoniak erfolgen, indem man beispielsweise das als Zwischenprodukt erhaltene 4-Hydroxydiphenylamin durch den oben beschriebenen Reaktionsturm pumpt und dem Produkt vor Eintritt in die auf die jeweilige Reaktionstemperatur erhitzte Kontaktzone Ammoniak zudosiert. Das aus dem Reaktionsturm austretende Rohprodukt wird entspannt, abgekühlt und durch Destillation aufgearbeitet.

In einer besonders bevorzugten Ausführungsform wird jedoch in zwei hintereinander geschalteten Reaktionstürmen gearbeitet. In dem ersten Reaktionsturm wird die Umsetzung des Hydroxybenzolderivates mit dem Anilinderivat durchgeführt und das austretende Gemisch von 4-Hydroxy-diphenylamin und gegebenenfalls Anilin ohne Zwischenisolierung in den zweiten nachgeschalteten Reaktionsturm gedrückt. Vor dem Eintritt in den zweiten Reaktionsturm wird das Ammoniak so zudosiert, daß 4-Hydroxydiphenylamin, Anilin und Ammoniak im Gleichstrom durch den γ-Aluminiumoxidkatalysator strömen. Die Reaktionstemperatur des ersten Reaktors beträgt bevorzugt 230 bis 280 °C, die des zweiten Reaktors bevorzugt 330 bis 380 °C. Der Druck in den Reaktoren kann je nach Art der Reaktanden und dem Molverhältnis in weiten Grenzen variieren. Er kann beispielsweise durch Zugabe von Inertgas gegebenenfalls noch erhöht werden.

Nach dem erfindungsgemäßen Verfahren erhält man ein Reaktionsprodukt, das direkt durch Destillation aufgearbeitet werden kann. Bei der Aufarbeitung fallen keine anorganischen Bestandteile an ; gegebenenfalls auftretende Rückstände können ohne Umweltbelastung gegebenenfalls verbrannt werden. Ein gegebenenfalls eingesetztes Lösungsmittel kann zurückgewonnen und wiederverwendet werden. Nach dem erfindungsgemäßen Verfahren erhält man 4-Amino-diphenylamin-Derivate der Formel

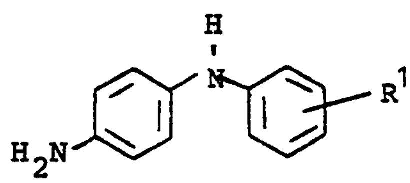

(IV)

worin

$R^1$ die obengenannte Bedeutung hat.

Es war nicht zu erwarten, daß nach dem erfindungsgemäßen Verfahren 4-Amino-diphenylaminderivate entstehen. Das erfindungsgemäße Verfahren ist überraschend, da bekannt war, daß die Umsetzung von Hydrochinon mit Ammoniak an einem γ-Aluminiumoxid-haltigen Katalysator zu p-Phenylendiamin und nicht zu dem entsprechenden 4-Aminophenol führt (JA 46/23053, JA 49/14737 und JA 49/29176). Ebenso führt die Aminierung von Hydrochinon mit Anilin an Molybdänoxid oder Nickeloxid auf Aluminiumoxid/Siliciumdioxid-Trägern zu N,N'-Diphenyl-1,4-diaminobenzol und nicht zu dem 4-Hydroxy-diphenylamin (US 3 510 518).

Nach dem erfindungsgemäßen Verfahren erhält man vorteilhafterweise in hohen Ausbeuten und mit großer Selektivität die 4-Amino-diphenylaminderivate. Eine Belastung der Umwelt durch Nebenprodukte tritt nicht auf.

Amino-diphenylamine können als Zwischenprodukte für die Herstellung von Bis-(4-anilino-phenoxy)-ester verwendet werden, die beispielsweise als Alterungsschutzmittel bei polymeren Verbindungen eingesetzt werden (DE-OS 2 748 205, DE-OS 2 753 194, DE-OS 2 801 414).

Beispiel 1

In einem kalibrierten Meßgefäß werden 396 g (3,6 Mol) Hydrochinon in 1338 g (14,4 Mol) Anilin gelöst. Diese Lösung wird über eine Stahlleitung mit einer Geschwindigkeit von 66 g Hydrochinon (0,6 Mol/h) und 223 g Anilin (2,4 Mol/h) in einen auf 270 °C erwärmten Stahlreaktor von 160 cm Höhe und 2,5 cm Innendurchmesser gepumpt, der mit 800 ml $\gamma$-$Al_2O_3$-Stücken von 1 cm Durchmesser und 1 cm Länge gefüllt ist. Dem Reaktor ist eine elektrisch beheizte Vorwärmzone vorgeschaltet. Das Reaktionsgemisch steht unter einem Druck von 20 bar. Das aus dem Ventil austretende Reaktionsprodukt wird in einem mit Siliconöl beschickten Stahlkühler kondensiert und in einem Vorratsgefäß gesammelt. Von dort wird es von einem konstanten Niveau aus in ein zweites, auf 350 °C beheiztes Rohr gleicher Dimensionierung und Füllung gepumpt. Dem Gemisch werden mit einer Hochdruckpumpe 194 g/h (12 Mol) Ammoniak zudosiert. Der Druck am Kopf des Reaktors ist auf 250 bar eingestellt. Das aus dem Ventil austretende Reaktionsgemisch wird gekühlt, kondensiert und in einer 2,5 l-Vorlage gesammelt. Das Ammoniak wird in ein mit Wasser gefülltes Überlaufgefäß geleitet und nicht isoliert.

Isoliertes Reaktionsprodukt pro Stunde : 277 g.

Das überschüssige Anilin wird bei 20 mbar abdestilliert. Es verbleibt ein Rückstand von 110 g mit einem Gehalt an 4-Aminodiphenylamin von 86,5 g (78,6 Mol-%), bezogen auf eingesetztes Hydrochinon.

Ausbeute : 78,6 % d.Th. bezogen auf eingesetztes Hydrochinon.

Beispiel 2

In einem 0,7 l-Stahlautoklaven werden 27 g Hydrochinon (0,25 Mol), 186 g Anilin (2,0 Mol) und 81 g $\gamma$-$Al_2O_3$-Kontakt DC-10 vorgelegt. Der Autoklav wird mit Stickstoff gespült und 2 Stunden bei 270 °C gerührt (Druck ca. 20 bar). Nach dem Abkühlen auf Raumtemperatur drückt man 85 g (5,0 Mol) Ammoniak auf und rührt 6 Stunden bei 350 °C. Es stellt sich ein Druck von 250 bis 300 bar ein.

Fünf dieser Ansätze werden vereinigt, die flüssigen Bestandteile von Katalysator abgesaugt und der Nutschrückstand dreimal mit 1 l DMF 1 Stunde bei 50 °C abgetrennt. Die organischen Filtrate werden vereinigt und eingeengt. Es verbleiben 1098 g an Rohlösung.

Die Lösung wird dann bei 20 mbar und 150 °C Sumpftemperatur bis zu einer Kopftemperatur von 150 °C vom restlichen Anilin und DMF befreit und anschließend unter Stickstoff im Hochvakuum bei 0,5 mbar bis zu einer Sumpftemperatur von 300 °C destilliert.

Man erhält 204,5 g (Kp. bis 200 °C) Destillat, das in der Vorlage erstarrt, = (88,9 Mol-%) und 9,6 g = (4,2) Gew.-% Rückstand, bezogen auf die theoretische Ausbeute an 4-Aminodiphenylamin von 230 g (1,25 Mol), bezogen auf eingesetztes Hydrochinon. Die Reinheit, bezogen auf 4-Aminodiphenylamin, beträgt 88,1 %. Daraus folgt eine Ausbeute von 78,3 % der theoretischen Ausbeute.

Beispiele 3 bis 5

Analog zu Beispiel 2 wurden die Beispiele 3 bis 6 durchgeführt, nur daß anstelle von reinem $\gamma$-$Al_2O_3$ dotiertes $\gamma$-$Al_2O_3$ der angegebenen Zusammensetzung verwendet wurde :

| Beispiel | Katalysator | AusbeuteMol-% 4-Aminodiphenylamin |
|---|---|---|
| 3 | $\gamma$-$Al_2O_3/WO_3/P_2O_5$ | 69,3 |
| 4 | $\gamma$-$Al_2O_3/MoO_3$ | 63,0 |
| 5 | $\gamma$-$Al_2O_3/SiO_2$ | 49,3 |

Beispiel 6

In einem 700 ml-Stahlautoklaven werden 108 g (etwa 1,0 Mol) Hydrochinon, 108 g zerkleinerter $\gamma$-Aluminiumoxid-Katalysator und 186 g (2,0 Mol) Anilin 4 Stunden bei 270 °C in Stickstoffatmosphäre gerührt. Der Druck beträgt 20 bar. Das helle, feste Reaktionsprodukt wird mit Methylenchlorid in einem Soxlethextraktor 16 Stunden extrahiert. Das Filtrat wird im Ölpumpenvakuum destilliert.

Vorlauf : 84 g $Kp_{2,7}$ mbar → 167° Sumpf
→ 71° Kopf
Hauptlauf : 166 g $Kp_{1,7}$ mbar → 300° Sumpf
→ 203° Kopf
Rückstand : 6 g
256 g

Analyse des Destillats : 94,7 % 4-Hydroxydiphenylamin.

Daraus ergibt sich für 4-Hydroxydiphenylamin eine Ausbeute von 85,0 % der theoretischen Ausbeute.

Beispiel 7

In einem 700 ml-Stahlautoklaven werden 108 g (etwa 1,0 Mol) Hydrochinon, 108 g γ-Aluminiumoxid-Katalysator und 215 g o-Toluidin (2,0 Mol) 3 Stunden bei 270 °C in Stickstoffatmosphäre gerührt. Der Druck beträgt 6 bar. Die Aufarbeitung erfolgt analog Beispiel 7.

| | | |
|---|---|---|
| Destillat : | 170 g = (85,9 %) | Fp 84-88° $Kp_{1,3}$ ansteigend bis 215 °C |
| Destillationsrückstand : | 12 g = (6,1 %) | |
| Rückstand in Katalysator : | 10 g = (5,1 %) | |
| | 192 g = (97,5 %) | |

Analyse des Destillats : 95,3 % = (81,8 Mol-%) 4-Hydroxy-2'-methyldiphenylamin.

Beispiel 8

In einem 700 ml-Stahlautoklaven werden 108 g (etwa 1,0 Mol) Hydrochinon, 108 g γ-Aluminiumoxid-Katalysator und 215 g (etwa 2,0 Mol) m-Toluidin 3 Stunden bei 270 °C unter Stickstoff gerührt. Der Druck beträgt 19,1 bar. Die Aufarbeitung des Reaktionsgemisches erfolgt analog Beispiel 7.

| | | |
|---|---|---|
| Destillat : | 172,5 g = (87,1 %) | Fp 88-92° $Kp_{0,7}$ 235° |
| Destillationsrückstand : | 12,0 g = (6,1 %) | |
| Rückstand im Katalysator : | 9,5 g = (4,6 %) | |
| | 194,0 g = (98,3 %) | |
| theoretisch : | 197,0 g = (100 %) | |

Analyse des Destillats : 95,3 % 4-Hydroxy-3'-methyldiphenylamin.
Ausbeute : 83,0 % (der theoretischen Ausbeute)

Beispiel 9

In einem 700 ml-Stahlautoklaven werden 108 g Hydrochinon (etwa 1,0 Mol), 215 g p-Toluidin (etwa 2,0 Mol) und 108 g γ-Aluminiumoxid-Katalysator 4 Stunden bei 270 °C unter Stickstoff gerührt. Der Druck beträgt 19 bar. Die Aufarbeitung des Reaktionsgemisches erfolgt analog Beispiel 7.
Destillat : 266 g (enthält p-Toluidin)
Destillationsrückstand : 18 g = (9,1 %)
Rückstand im Katalysator : 8 g = (4,1 %)
Analyse des Destillats : 34,2 % p-Toluidin
60,6 % 4-Hydroxy-4'-methyldiphenylamin
Ausbeute : 81,4 % (der theoretischen Ausbeute)
Fp 120-122° nach Umkristallisation aus Toluol.

Beispiel 10

In einem 700 ml-Stahlautoklaven werden 45 g 4-Hydroxydiphenylamin (etwa 0,24 Mol), 88 g γ-Aluminiumoxid-Katalysator und 120 g $NH_3$ (7,0 Mol) unter Stickstoff 2 Stunden bei 350 °C gerührt. Es entsteht ein Druck von 395 bar. Insgesamt werden 8 Versuche durchgeführt. Der Druck beträgt 350 bis 395 bar. Jeder Versuch wird mit DMF extrahiert. Die vereinten DMF-Lösungen werden unter Stickstoff destilliert. Die Hauptmenge des Reaktionsprodukts destilliert bei 2,0 mbar bei 174 bis 177 °C. Die Kopftemperatur steigt gegen Ende der Destillation auf 210 °C.
Destillat : 305 g = (85,2 %)
Destillationsrückstand : 21 g = (5,9 %)
Analyse nach der Destillation : 96,4 % 4-Aminodiphenylamin
Ausbeute : 82,1 % (der theoretischen Ausbeute).

Beispiel 11

In der gleichen Weise wie in Beispiel 11 werden 4-Hydroxy-2'-methyl-, 4-Hydroxy-3'-methyl- und 4-Hydroxy-4'-methyldiphenylamin mit Ammoniak bei 350 °C unter Stickstoff in 2 Stunden umgesetzt.
4-Amino-2'-methyldiphenylamin
Druck 272 bar Ammoniak : 85 g = (5,0 Mol)
Ausbeute 4-Amino-2'-diphenylamin : 70,7 % der theoretischen Ausbeute.

Beispiel 12

4-Amino-3'-methyldiphenylamin
Druck 260 bar   Ammoniak : 85 g = (5,0 Mol)
Ausbeute 4-Amino-3'-diphenylamin : 86,1 % der theoretischen Ausbeute.

Beispiel 13

4-Amino-4'-methyldiphenylamin
Druck 250 bar   Ammoniak : 85 g = (5,0 Mol)
Ausbeute 4-Amino-4'-methyldiphenylamin : 82,2 % (der theoretischen Ausbeute).

Biespiel 14

27 g 4-Aminophenol (0,25 Mol), 54 g $\gamma$-$Al_2O_3$-Katalysator und 186 g Anilin (2,0 Mol) werden unter Stickstoff in einem 0,7 l-Stahlautoklaven 3 Stunden bei 350 °C gerührt. Der Druck beträgt 20 bar. Man kühlt ab, drückt 120 g Ammoniak (etwa 7,0 Mol) auf und rührt weitere 4 Stunden bei 350 °C. Der dunkle schmierige Rückstand wird unter Stickstoff bei Raumtemperatur mit 2 DMF gerührt, der Katalysator abfiltriert und das Filtrat gaschromatographisch untersucht.
Ausbeute : 29,3 % (der theoretischen Ausbeute).

Beispiele 15 und 16

In analoger Weise zu Beispiel 15 wurden die Versuche 16 und 17 durchgeführt, nur daß anstelle von reinem $\gamma$-$Al_2O_3$ der jeweilige dotierte $\gamma$-$Al_2O_3$-haltige Katalysator verwendet wurde.

| Beispiel | Katalysator | Ausbeute (bezogen auf das eingesetzte 4-Aminophenol) Mol-% 4-Aminodiphenylamin |
|---|---|---|
| 15 | $\gamma$-$Al_2O_3/MoO_3$ | 27,8 |
| 16 | $\gamma$-$Al_2O_3/WO_3/P_2O_5$ | 37,2 |

**Ansprüche**

1. Verfahren zur Herstellung von 4-Amino-diphenylaminoderivaten, dadurch gekennzeichnet, daß man in Gegenwart eines $\gamma$-Aluminiumoxid enthaltenden Katalysators Hydroxybenzolderivate der Formel

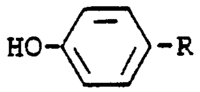
(I)

worin
R Hydroxy oder Amino bedeutet,
mit einem Anilinderivat der Formel

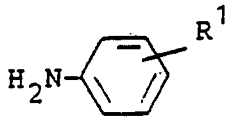
(II)

worin
$R^1$ für Wasserstoff oder einen Alkylrest steht,
umsetzt und anschließend am gleichen Katalysator mit Ammoniak behandelt.

2. Verfahren nach Anspruch 1, dadurch gekennzeichnet, daß man undotiertes $\gamma$-Aluminiumoxid einsetzt.

3. Verfahren nach den Ansprüchen 1 und 2, dadurch gekennzeichnet, daß man im Temperaturbereich von 200 bis 450 °C arbeitet.

4. Verfahren nach den Ansprüchen 1 bis 3, dadurch gekennzeichnet, daß man im Druckbereich von 1 bis 400 bar arbeitet.

5. Verfahren nach den Ansprüchen 1 bis 4, dadurch gekennzeichnet, daß man Hydrochinon im Temperaturbereich von 230 bis 280 °C mit 1 bis 10 Mol eines Anilinderivates der Formel II umsetzt und dann im Temperaturbereich von 330 bis 380 °C am gleichen Katalysator mit 15 bis 30 Mol Ammoniak behandelt.

6. Verfahren nach den Ansprüchen 1 bis 4, dadurch gekennzeichnet, daß man 4-Amino-phenol mit Anilin im Temperaturbereich von 330 bis 380 °C umsetzt und das entstehende Gemisch aus 4-Hydroxy- und 4-Amino-diphenylamin im gleichen Temperaturbereich am gleichen Katalysator mit 15 bis 30 Mol Ammoniak behandelt.

7. Verfahren nach den Ansprüchen 1 bis 6, dadurch gekennzeichnet, daß man in überschüssigem Anilinderivat als Lösungsmittel arbeitet.

**Claims**

1. Process for the preparation of 4-amino-diphenylamino derivatives, characterised in that hydroxybenzene derivatives of the formula

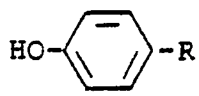

(I)

wherein

R denotes hydroxyl or amino,

are reacted with an aniline derivative of the formula

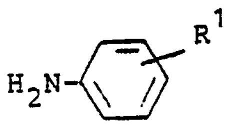

(II)

wherein

$R^1$ represents hydrogen or an alkyl radical, in the presence of a catalyst containing $\gamma$-aluminium oxide, and the product is then treated with ammonia on the same catalyst.

2. Process according to Claim 1, characterised in that undoped $\gamma$-aluminium oxide is employed.

3. Process according to Claims 1 and 2, characterised in that it is carried out in the temperature range from 200 to 450 °C.

4. Process according to Claims 1 to 3, characterised in that it is carried out in the pressure range from 1 to 400 bars.

5. Process according to Claims 1 to 4, characterised in that hydroquinone is reacted with 1 to 10 mols of an aniline derivative of the formula II in the temperature range from 230 to 280 °C and then the mixture is treated with 15 to 30 mols of ammonia in the temperature range from 330 to 380 °C on the same catalyst.

6. Process according to Claims 1 to 4, characterised in that 4-amino-phenol is reacted with aniline in the temperature range from 330 to 380 °C and the resulting mixture of 4-hydroxy- and 4-amino-diphenylamine is treated with 15 to 30 mols of ammonia in the same temperature range on the same catalyst.

7. Process according to Claims 1 to 6, characterised in that it is carried out in excess aniline derivative as the solvent.

**Revendications**

1. Procédé de fabrication de dérivés de 4-amino-diphénylamine, caractérisé en ce qu'on fait réagir, en présence d'un catalyseur contenant de l'oxyde d'aluminium gamma, des dérivés d'hydroxybenzène de formule :

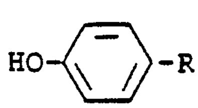

(I)

dans laquelle

R signifie un hydroxy ou amino,

avec un dérivé d'aniline de formule :

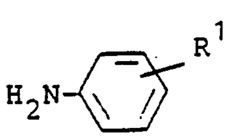

(II)

dans laquelle

$R^1$ représente de l'hydrogène ou un radical alcoyle, puis en ce qu'on les traite sur le même catalyseur avec de l'ammoniac.

2. Procédé selon la revendication 1, caractérisé en ce qu'on utilise de l'oxyde d'aluminium gamma non doté.

3. Procédé selon les revendications 1 et 2, caractérisé en ce qu'on opère dans l'intervalle de température de 200 à 450 °C.

4. Procédé selon les revendications 1 à 3, caractérisé en ce qu'on opère dans l'intervalle de pression de 1 à 400 bars.

5. Procédé selon les revendications 1 à 4, caractérisé en ce qu'on fait réagir de l'hydroquinone dans l'intervalle de température de 230 à 280 °C avec 1 à 10 moles d'un dérivé d'aniline de formule II, puis en ce qu'on le traite dans l'intervalle de température de 330 à 380 °C sur le même catalyseur avec 15 à 30 moles d'ammoniac.

6. Procédé selon les revendications 1 à 4, caractérisé en ce qu'on fait réagir du 4-amino-phénol avec de l'aniline dans l'intervalle de température de 330 à 380 °C et en ce qu'on traite le mélange obtenu de 4-hydroxy- et 4-amino-diphénylamine dans le même intervalle de température et sur le même catalyseur avec 15 à 30 moles d'ammoniac.

7. Procédé selon les revendications 1 à 6, caractérisé en ce qu'on opère dans un excès de dérivé d'aniline en tant que solvant.